(19) **Europäisches Patentamt / European Patent Office / Office européen des brevets**

(11) **EP 3 146 906 A1**

(12) **EUROPEAN PATENT APPLICATION**
published in accordance with Art. 153(4) EPC

(43) Date of publication:
**29.03.2017 Bulletin 2017/13**

(51) Int Cl.:
***A61B 17/02*** (2006.01)

(21) Application number: **15796741.5**

(22) Date of filing: **15.05.2015**

(86) International application number:
**PCT/JP2015/064042**

(87) International publication number:
**WO 2015/178311 (26.11.2015 Gazette 2015/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **19.05.2014 JP 2014103701**

(71) Applicant: **Toray Fine Chemicals Co., Ltd.**
**Tokyo 101-0041 (JP)**

(72) Inventors:
• **KATAOKA, Shintaro**
  **Matsuyama**
  **Ehime 791-8057 (JP)**
• **HIRABARA, Takehiko**
  **Osaka 530-0017 (JP)**

(74) Representative: **Kador & Partner**
**Corneliusstraße 15**
**80469 München (DE)**

(54) **EXCLUDER**

(57) Provided is a retractor for endoscopic surgery and the like, which can be placed in a vicinity of a target organ in a body cavity, while reducing burdens on a patient. The retractor is a surgical retractor including a liquid-absorbing and swelling material, wherein the liquid-absorbing and swelling material is formed in a roll shape, the roll-shaped liquid-absorbing and swelling material absorbs liquid in a body cavity to swell and expand into a planar shape.

FIG. 1A

## FIG. 1B

## FIG. 1C

**Description**

TECHNICAL FIELD

The present invention relates to a retractor placed in a vicinity of a target organ (surgical object organ) during surgery.

BACKGROUND ART

Conventional surgery typically requires a long incision made on an abdomen or a thorax. Therefore, making such a long incision involves risks such as reopening of the incision site and development of infection. In addition, a patient must experience mental pain because of not being able to move his body, and suffers from pain due to the incision, as well as he must endure for long periods of time to recover after the surgery, and the scar would not disappear. However, by virtue of the development of the medical technologies, medical instruments, and medical devices, endoscopic (laparoscope) surgery has frequently been taken in recent years. In endoscopic surgery, a medical instrument (trocar) is inserted into an abdominal cavity through a small opening made on the abdomen, and the medical device is manipulated with the support of monitoring device. The endoscopic surgery reduces the mental pain given to a patient during surgery.

In endoscopic surgery, it is desirable that other organs which may interfere in the visibility are retracted to ensure that the target organ becomes visible. Therefore, retractors for endoscopic surgery, which can be inserted into a body cavity through a trocar, and expand the interior of body cavity so as to retract non-targeted organs, are proposed (for example, see PTLs 1, 2). However, these retractors must be supported by a person during insertion, and require an opening made on a patient's body for supporting the retractor.

To solve these problems, a retractor including a bar-shaped water-absorbing and swelling material having a cross section smaller than the cross section of the inner cavity of the trocar, in which the water-absorbing and swelling material is obtained by drying and compression molding, is proposed (for example, see PTL 3).

CONVENTIONAL ART DOCUMENTS

PATENT DOCUMENTS

Patent Document 1: JP 2003-164459 A
Patent Document 2: JP 2005-253916 A
Patent Document 3: JP 5128672 B

SUMMARY OF THE INVENTION

PROBLEMS TO BE SOLVED BY THE INVENTION

The evolution in endoscopic surgery aiming to reduce the size of opening made on the patient's body during surgery, whereby reducing burdens on a patient, is still in progress. Therefore, there is a demand for downsizing medical instruments and medical devices. However, a retractor having a reduced cross section may bring a situation as follows. That is, a bar-shaped water-absorbing and swelling material, which is obtained by the drying and compression molding, has a limit of its swelling ratio by absorbing water. Therefore, the bar-shaped water-absorbing and swelling material may be readily inserted into a body cavity, but swelling after the insertion may not provide a sufficient size for retracting organs.

The object of the present invention is to provide a retractor for endoscopic surgery and the like, which can be placed in a vicinity of a target organ in a body cavity, while reducing burdens on a patient.

MEANS TO SOLVE THE PROBLEMS

In order to achieve the object, the retractor of the present invention is a surgical retractor including a liquid-absorbing and swelling material, wherein the liquid-absorbing and swelling material is formed in a roll shape, the roll-shaped liquid-absorbing and swelling material absorbs liquid in a body cavity to swell and expand into a planar shape.

The roll-shaped liquid-absorbing and swelling material is preferably formed by rolling a sheet-shaped liquid-absorbing and swelling material into a roll shape.

The sheet-shaped liquid-absorbing and swelling material is preferably compressed in the thickness direction.

The roll-shaped liquid-absorbing and swelling material preferably expands, in a roll direction, by 8 to 42 times the diameter of the roll.

The roll-shaped liquid-absorbing and swelling material preferably lifts and retracts an organ in a vicinity of a target.

EFFECTS OF THE INVENTION

According to the present invention, provided is a retractor for endoscopic surgery and the like, which can be placed in a vicinity of a target organ in a body cavity, while reducing burdens on a patient.

BRIEF DESCRIPTION OF THE DRAWINGS

[FIG. 1] FIGs. 1A, 1B, and 1C show an exemplary embodiment of a retractor of the present invention. FIG. 1A is a plan view of the retractor of the present invention, FIG. 1B is a perspective view of the retractor shown in FIG. 1A when viewed from the lateral side, and FIG. 1C is a plan view when the retractor is expanded.

[FIG. 2] FIG. 2 is a schematic view showing an exemplary method of producing the retractor of the present invention.

EMBODIMENTS FOR CARRYING OUT THE INVENTION

The retractor of the present invention will be described with reference to examples. It is noted that the present invention is not limited and restricted to the following examples. It is further noted that figures referred in the following description are represented in a schematic manner, and thus, a scale of object drawn in a figure may differ from that of the actual object and the like. The scales of objects and the like may differ between respective figures.

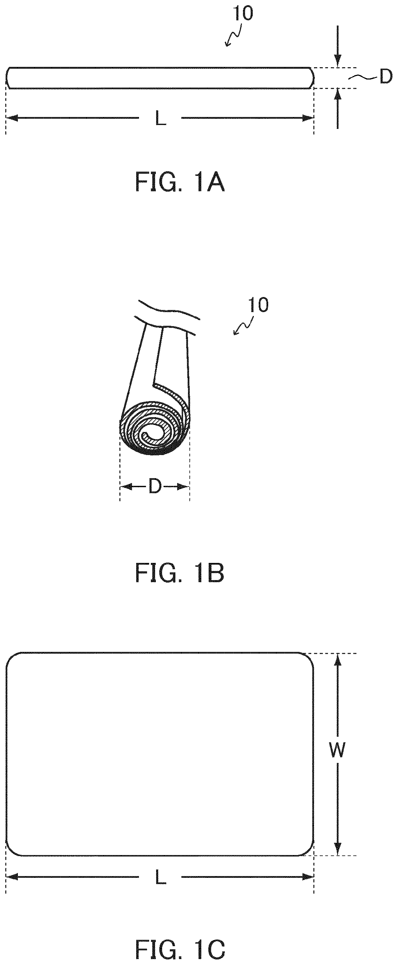

FIGs. 1A, 1B, and 1C show a retractor 10 according to an embodiment of the present invention. FIG. 1A is a plan view of the retractor 10, FIG. 1B is a perspective view of the retractor 10 shown in FIG. 1A when viewed from the lateral side, and FIG. 1C is a plan view when the retractor 10 is expanded. The retractor 10 is a surgical retractor suitable for use in endoscopic surgery and the like, and includes a liquid-absorbing and swelling material. The liquid-absorbing and swelling material is formed in a roll shape, which has a diameter of D and a length of L. The roll-shaped liquid-absorbing and swelling material can swell by absorbing liquid in a body cavity, and expand into a planar shape, which has a widthwise direction of W, thereby being placed in a vicinity of a target organ. The retractor expanding on a plane in the vicinity of the target organ prevents surgical devices from contacting organs around the target organ to protect these organs around the target organ. The retractor can also lift and retract the organs around the target organ.

Preferably, the liquid-absorbing and swelling material that is formed in a sheet shape is compressed in the thickness direction. This compression can be achieved by drying and compression process, in which the liquid-absorbing and swelling material is dried, and then compressed in the thickness direction. When the retractor formed by rolling the compressed sheet-shaped liquid-absorbing and swelling material absorbs liquid, the sheet expands into a planar shape (returns from the roll shape into the planar shape) and swells in the thickness direction. For this reason, even when the opening for inserting the retractor into the body cavity is small, the retractor can readily be inserted, and swell largely by absorbing liquid in the body cavity, allowing more effective protection of the organs around the target organ, and lifting and retraction of the organs around the target organ, while reducing burdens on a patient.

In such retractor, the compression rate is preferably 20% or less, more preferably 15% or less. Such compression rate can be expected to provide excellent swelling performance when providing water. In this context, a compression rate (%) is calculated by the following formula: (the size in the compression direction after drying and compression molding) / (the size in the corresponding direction when produced without compression) $\times$ 100.

Preferably, the retractor 10 according to the present invention has a swelling ratio in the compression direction by absorbing liquid of 5 times or more. In particular, for the purpose of effective lifting and retraction of organs, 10 times or more is more preferable. Such swelling ratio allows a large amount of swelling sufficient for providing excellent protection for organs as well as effective lifting and retraction.

Preferably, the liquid-absorbing and swelling material of the present invention is a cellulose porous body such as cellulose sponge. This is because cellulose has excellent safety for a biological body. The cellulose sponge material, which can be processed by the drying and compression molding, is a preferable material in that the material swells by absorbing the water when the dried and compressed cellulose sponge is provided with water.

As a cellulose sponge suitable for use in the present invention, cellulose sponges produced by conventional producing processes, such as the regenerated cellulose method or cellulose solvent solution method (for example, a cellulose sponge disclosed in JP 3520511) can be used without any additional treatment. Specifically, viscose added with natural fibers is prepared from a dissolving pulp containing cellulose as a main component. Crystaline mirabilite is added and mixed to the viscose to prepare a mixture. A block-shaped, or sheet-shaped cellulose sponge can be obtained by pressing the mixture into a mold or ejecting the mixture into a sheet shape, and then thermally coagulating the sheet-shaped mixture. Further, it also preferable that cotton, flax, ramie, or pulp is used alone or in combination as a reinforcing fiber for the cellulose sponge. Inclusion of these reinforcing fibers makes the sponge stronger, and suppresses the generation of lint and breakage and dropping out of the retractor when the sponge is removed from the trocar after surgery.

Examples of commercially available cellulose sponges include Toray cellulose sponges (manufactured by Toray Fine Chemicals Co., Ltd., trade name) and the like. This original fabric of cellulose sponge, which has a block shape, for example, can be cut or punched in a size of a cellulose sponge to be used as the liquid-absorbing and swelling material of the present invention.

There is no need for a special postprocessing and the like to provide water-absorbing property for cellulose sponges, because cellulose itself has a water-absorbing property, and thus, it is possible to suppress a cost increase caused by an increased number of steps of postprocessing or risk management of safety of chemicals used in the postprocessing. Further, cellulose sponges have an excellent handling property during surgery because of its low lint generation, and the sponges are readily removed after surgery due to a significantly low adherence property to tissues of the incision site. When a cellulose sponge is inserted between an organ that is a surgical object and organs therearound in order to retract the organs and obtain a field of operation during surgery, its liquid-absorbing and swelling property also provides benefits of protecting organs, absorbing blood or body fluids and the like.

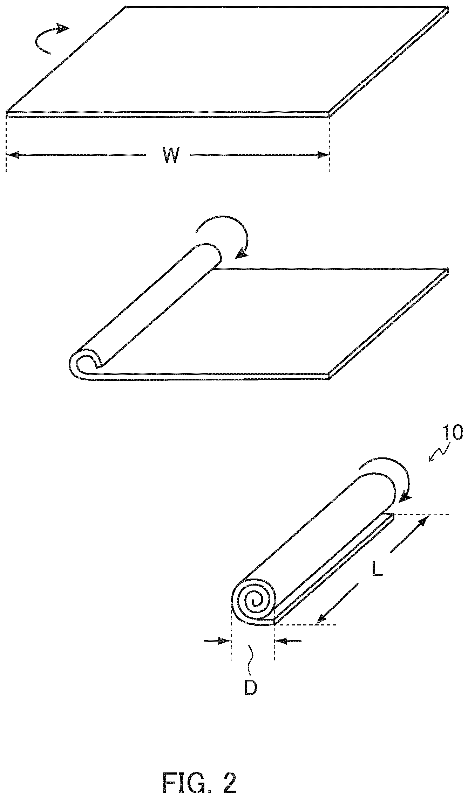

A material preformed in a roll shape may be used as the roll-shaped liquid-absorbing and swelling material. However, as shown in FIG. 2, it is preferable that a material formed in a sheet shape is rolled to form the roll-shaped liquid-absorbing and swelling material. More preferably, the liquid-absorbing and swelling material formed in a sheet shape is compressed in the thickness direction, and then formed into a roll shape. This forming procedure allows the liquid-absorbing and swelling material to increase its volume, when impregnated with physiological saline and the like, by 1.5 to 2.5 times compared to a conventional liquid-absorbing and swelling material that is only compressed. In endoscopic surgery, the shape or outer diameter of a retractor to be inserted depends on the inner diameter of a trocar. Thus, expanding a retractor having a small diameter during the insertion into a shape having a wider area in the body cavity provides the ability of alleviating restrictions due to the body type of the patient, which is preferable. Further, when a volume of the retractor required in a body cavity is already determined, a smaller size of the retractor before insertion is preferable, because it means that the diameter of the trocar can be downsized, thereby it can be expected to reduce burdens on a patient after surgery. For example, assuming that the volume (required dimensions) of the retractor in the body cavity is 300 mm in the longitudinal direction, 80 mm in the lateral direction, and 8 mm in the thickness direction, even when the lateral length (80 mm) is compressed into 8 mm (1/10, that is almost equal to the compression limit), the length of the diagonal line of the compressed cross section (a rectangle) of the retractor is 11.3 mm, because the shape of the compressed cross section by compression molding is limited to a rectangle. On the other hand, when the retractor having the above required dimensions is formed into a roll shape, its thickness (8 mm) can be compressed to 1/10 to 1/11 (0.8 to 0.7 mm) and then the retractor can be formed in a roll shape. Therefore, after the roll formation, the retractor can be in a more compact form having a circular cross section of $\phi$7.5 mm.

As such, when a cellulose sponge is used as a sheet-shaped liquid-absorbing and swelling material, the cellulose sponge impregnated with physiological saline and the like preferably has a thickness in a range of 15 to 3 mm, more preferably in a range of 10 to 5 mm. In these thickness ranges, for example, the thickness of 9 mm may result in a range of 0.7 to 0.6 mm, and the thickness of 5 mm may result in a range of 0.5 to 0.4 mm, after the drying and compression process, and thus, the handling property during the rolling process can be excellent.

Since the present retractor is formed into a roll shape, the liquid-absorbing and swelling material can be fixed with a mold such as a resin mold. Thus, various shapes such as a rectangle, polygon, circle, C-shape and a concaved shape may be provided as the cross section of the mold depending on the shape of the insertion hole of the trocar.

When the retractor formed in a roll shape is swelled in a body cavity by dropping, for example, physiological saline, the retractor swells and expands gradually from the formed roll shape into a sheet shape, regardless of where the dropping occurs, as long as a required amount of water is retained. Therefore, there is no directional restriction during insertion in order to recover the dimension before the formation process. An amount of physiological saline required for swelling the retractor in a body cavity may be a water amount corresponding to the same volume as the volume of the retractor before forming the roll (or before the compression process, where the roll shape is formed after the compression). When water is provided to the retractor in an amount greater than such water amount, the retractor cannot absorb the water, which is not preferable. Further, the retractor of the present invention can be formed into a thin sheet such that retraction appropriate for the internal situation, for example, protection by inserting a retractor into a narrow space between organs, or retraction with a retractor having a folded portion so as to increase its thickness for the purpose of increasing lifting effect, can be performed.

In addition, when being removed, the present retractor may be cut into strips which are thinner than the inner diameter of the trocar and then removed, since the retractor after surgery expands, in a roll direction (direction W in FIG. 1), by 8 to 42 times the roll diameter D before surgery. In this context, when the retractor has different values of tensile strength in longitudinal, lateral, and thickness directions, respectively, a direction having a high tensile strength is employed beforehand as the longitudinal direction during the processing of the retractor, for example, and thereby a safer retractor in which breaking, separation and the like during removal are suppressed can be formed.

For example, when a sheet is produced from a block-shaped cellulose sponge, employing Z-axis direction as the extruding direction of manufacturing process may result in reduced tensile strength in one of the directions orthogonal to Z-axis. When such direction is employed as Y-axis direction, and the direction orthogonal to Z-axis direction and Y-axis direction is employed as X-axis direction, employing Y-axis direction as thickness direction results in providing a tensile-resistant sheet. Measured values of tensile strength of the block-shaped cellulose sponge are 9 to 17 N/cm$^2$ in X-axis direction, 4 to 9 N/cm$^2$ in Y-axis direction, and 9 to 18 N/cm$^2$ in Z-axis direction, for example. These tensile strength values are averaged values of measured tensile strength values (N/cm$^2$) in a tensile test for 10 or more test pieces having a size of 7 cm × 2 cm × 1 cm, in which Tensilon universal testing machine is used, the direction having the length of 7 cm is employed as an axial direction along which tension force is applied, and a chuck-to-chuck distance is set to 5 cm.

Further, the retractor of the present invention may include X-ray contrast thread. The X-ray contrast thread may be placed in any place within the retractor as long as they provide contrast effect, but preferably placed in a location where mostly does not contact with organs. For example, the X-ray contrast fibers are preferably placed at edges or side portions of the planar portion in the form of a sheet or a line, and more preferably, the X-ray contrast fibers are placed in the longitudinal direction of the winding end of the winding sponge. The X-ray contrast thread is placed at any interval as long as they provides contrast effect, but it is more preferable that the X-ray contrast thread, that is forming a solid line, is placed along the entire length of the roll.

When X-ray opaque heat-fusible resin yarn is used as the X-ray contrast thread, the X-ray opaque heat-fusible resin yarn can be thermally bonded to the retractor. Examples of the X-ray opaque heat-fusible resin yarn include monofilaments or multifilaments of polypropylene-based resins kneaded with barium sulfate, yarns of vinyl chloride resin kneaded with barium sulfate and the like.

EXAMPLES

(Example 1)

A block-shaped cellulose sponge was prepared. Specifically, viscose added with natural fibers was prepared from a dissolving pulp containing cellulose as a main component, then a mixture was formed by adding crystaline mirabilite into the viscose. A block-shaped cellulose sponge was obtained by pressing the mixture into a mold and thermally coagulating it. The resulting block-shaped cellulose sponge was sliced and punched to produce a sheet-shaped cellulose sponge having a lateral length of 120 mm x longitudinal length of 180 mm × thickness of 3 mm, and then the cellulose sponge was dried to be a dried cellulose sponge.

The above-described dried cellulose sponge was rolled into a bar shape, in which the longitudinal direction thereof was the length direction, and the lateral direction thereof was the winding direction. The cellulose sponge was placed into a tube-shaped resin mold having a C-shaped cross section and the inner diameter of ϕ9 mm. A lid, which was a resin mold processed in an arc shape, was placed onto the exposed portion of the cellulose sponge, and the lid was fixed so as not to be detached. The rolled cellulose sponge within the resin mold was placed in a dryer at 60°C for 1 hour, then removed and left to cool down for at least 1 hour at room temperature. Thereafter, the cellulose sponge was removed from the resin mold. As a result, a roll-shaped retractor was obtained. The roll-shaped retractor had a diameter of ϕ10.8 mm (maximum value) when removed from the resin mold, and its expanding ratio was 11 times in the winding direction, and its exterior shape was a roll shape.

(Example 2)

The block-shaped cellulose sponge was cut and punched to produce a sheet-shaped cellulose sponge having a lateral length of 120 mm × longitudinal length of 180 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled into a bar shape, in which the longitudinal direction thereof was the length direction, and the lateral direction thereof was the winding direction. The cellulose sponge was placed into a tube-shaped resin mold having a C-shaped cross section and the inner diameter of ϕ9 mm. A lid, which was a resin mold processed in an arc shape, was placed onto the exposed portion of the cellulose sponge, and the lid was fixed so as not to be detached. The rolled cellulose sponge within the resin mold was placed in a dryer at 60°C for 1 hour, then removed and left to cool down for at least 1 hour at room temperature. Thereafter, the cellulose sponge was removed from the resin mold. As a result, a roll-shaped retractor was obtained. The roll-shaped retractor had a diameter of $\phi9.7$ mm (maximum value) when removed from the resin mold, and its expanding ratio was 12 times in the winding direction, and its exterior shape was a roll shape.

(Example 3)

The block-shaped cellulose sponge was cut and punched to produce a sheet-shaped cellulose sponge having a lateral length of 120 mm × longitudinal length of 180 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.

The above-described compressed cellulose sponge was rolled and dried as in Example 2 in order to obtain a roll-shaped retractor. The roll-shaped retractor had a diameter of $\phi10.3$ mm (maximum value) when removed from the resin mold, and its expanding ratio was 12 times in the winding direction, and its exterior shape was a roll shape.

(Example 4)

The block-shaped cellulose sponge was cut and punched to produce a sheet-shaped cellulose sponge having a lateral length of 120 mm × longitudinal length of 300 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled and dried as in Example 2 in order to obtain a roll-shaped retractor. The roll-shaped retractor had a diameter of $\phi10.1$ mm (maximum value) when removed from the resin mold, and its expanding ratio was 12 times in the winding direction, and its exterior shape was a roll shape.

(Example 5)

The block-shaped cellulose sponge was cut and punched to produce a sheet-shaped cellulose sponge having a lateral length of 120 mm × longitudinal length of 300 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.

The above-described compressed cellulose sponge was rolled and dried as in Example 2 in order to obtain a roll-shaped retractor. The roll-shaped retractor had a diameter of $\phi10.9$ mm (maximum value) when removed from the resin mold, and its expanding ratio was 12 times in the winding direction, and its exterior shape was a roll shape.

(Example 6)

The block-shaped cellulose sponge was cut and punched to produce a sheet-shaped cellulose sponge having a lateral length of 150 mm × longitudinal length of 180 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.

The above-described compressed cellulose sponge was rolled and dried as in Example 2 in order to obtain a roll-shaped retractor. The roll-shaped retractor had a diameter of $\phi10.0$ mm (maximum value) when removed from the resin mold, and its expanding ratio was 15 times in the winding direction, and its exterior shape was a roll shape.

(Example 7)

The block-shaped cellulose sponge was cut and punched to produce a sheet-shaped cellulose sponge having a lateral length of 100 mm × longitudinal length of 300 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled and dried as in Example 2 in order to obtain a roll-shaped retractor. The roll-shaped retractor had a diameter of $\phi10.0$ mm (maximum value) when removed from the resin mold, and its expanding ratio was 10 times in the winding direction, and its exterior shape was a roll shape.

(Example 8)

The block-shaped cellulose sponge was cut and punched to produce a sheet-shaped cellulose sponge having a lateral length of 80 mm × longitudinal length of 250 mm × thickness of 9 mm. Then the cellulose sponge was dried and compressed in the thickness direction at 130°C to be a compressed cellulose sponge. The compressed cellulose sponge had a thickness of 0.6 mm (maximum value), and the compression rate was 6.7%.

The above-described compressed cellulose sponge was rolled and dried as in Example 2 in order to obtain a roll-shaped retractor. The roll-shaped retractor had a diameter of $\phi$10.6 mm (maximum value) when removed from the resin mold, and its expanding ratio was 8 times in the winding direction, and its exterior shape was a roll shape.

(Example 9)

The block-shaped cellulose sponge was cut and punched to produce a sheet-shaped cellulose sponge having a lateral length of 120 mm × longitudinal length of 180 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled into a bar shape, in which the longitudinal direction thereof was the length direction, and the lateral direction thereof was the winding direction. Then, the cellulose sponge was placed into a tube-shaped resin mold having a C-shaped cross section and the inner diameter of $\phi$9 mm (the arc-shaped lid used in Example 2 was not used for this Example). The rolled cellulose sponge within the resin mold was placed in a dryer at 60°C for 1 hour, then removed and left to cool down for at least 1 hour at room temperature. Thereafter, the cellulose sponge was removed from the resin mold. As a result, a roll-shaped retractor was obtained. The roll-shaped retractor had a diameter of $\phi$9.7 mm (maximum value) when removed from the resin mold, and its expanding ratio was 12 times in the winding direction, and its exterior shape was a roll shape.

(Example 10)

The block-shaped cellulose sponge was cut and punched to produce a sheet-shaped cellulose sponge having a lateral length of 120 mm × longitudinal length of 180 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled into a bar shape, in which the longitudinal direction thereof was the length direction, and the lateral direction thereof was the winding direction. Then, the cellulose sponge was placed into a tube-shaped resin mold having a circularly-shaped cross section and the inner diameter of $\phi$9 mm and placed in a dryer at 60°C for 1 hour, then removed and left to cool down for at least 1 hour at room temperature. Thereafter, the cellulose sponge was removed from the resin mold. As a result, a roll-shaped retractor was obtained. The roll-shaped retractor had a diameter of $\phi$9.8 mm (maximum value) when removed from the resin mold, and its expanding ratio was 12 times in the winding direction, and its exterior shape was a roll shape.

(Example 11)

The block-shaped cellulose sponge was cut and punched to produce a sheet-shaped cellulose sponge having a lateral length of 120 mm × longitudinal length of 180 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.

The above-described compressed cellulose sponge was rolled into a bar shape, in which the longitudinal direction thereof was the length direction, and the lateral direction thereof was the winding direction. Then, the cellulose sponge was placed into a tube-shaped resin mold having a C-shaped cross section and the inner diameter of $\phi$8 mm. Thereafter, the sponge was dried as in Example 2 in order to obtain a roll-shaped retractor. The roll-shaped retractor had a diameter of $\phi$8.7 mm (maximum value) when removed from the resin mold, and its expanding ratio was 14 times in the winding direction, and its exterior shape was a roll shape.

(Example 12)

The block-shaped cellulose sponge was cut and punched to produce a sheet-shaped cellulose sponge having a lateral length of 100 mm × longitudinal length of 300 mm × thickness of 7 mm. Then the cellulose sponge was dried and compressed in the thickness direction at 130°C to be a compressed cellulose sponge. The compressed cellulose sponge had a thickness of 0.6 mm (maximum value), and the compression rate was 8.6%.

The above-described compressed cellulose sponge was rolled and dried as in Example 11 in order to obtain a roll-shaped retractor. The roll-shaped retractor had a diameter of $\phi$8.6 mm (maximum value) when removed from the resin mold, and its expanding ratio was 12 times in the winding direction, and its exterior shape was a roll shape.

(Example 13)

The block-shaped cellulose sponge was cut and punched to produce a sheet-shaped cellulose sponge having a lateral length of 100 mm x longitudinal length of 150 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.
The above-described compressed cellulose sponge was rolled into a bar shape, in which the longitudinal direction thereof was the length direction, and the lateral direction thereof was the winding direction. Then, the cellulose sponge was placed into a tube-shaped resin mold having a C-shaped cross section and the inner diameter of $\phi$7 mm. Thereafter, the sponge was dried as in Example 2 in order to obtain a roll-shaped retractor. The roll-shaped retractor had a diameter of $\phi$10.0 mm (maximum value) when removed from the resin mold, and its expanding ratio was 10 times in the winding direction, and its exterior shape was a roll shape.

(Example 14)

The block-shaped cellulose sponge was cut and punched to produce a sheet-shaped cellulose sponge having a lateral length of 120 mm × longitudinal length of 180 mm × thickness of 3 mm. Then the cellulose sponge was dried and compressed in the thickness direction at 130°C to be a compressed cellulose sponge. The compressed cellulose sponge had a thickness of 0.3 mm (maximum value), and the compression rate was 10.0%.
The above-described compressed cellulose sponge was rolled into a bar shape, in which the longitudinal direction thereof was the length direction, and the lateral direction thereof was the winding direction. Then, the cellulose sponge was placed into a tube-shaped resin mold having a C-shaped cross section and the inner diameter of $\phi$4 mm. Thereafter, the sponge was dried as in Example 2 in order to obtain a roll-shaped retractor. The roll-shaped retractor had a diameter of $\phi$4.8 mm (maximum value) when removed from the resin mold, and its expanding ratio was 25 times in the winding direction, and its exterior shape was a roll shape.

(Example 15)

The block-shaped cellulose sponge was cut and punched to produce a sheet-shaped cellulose sponge having a lateral length of 120 mm × longitudinal length of 460 mm × thickness of 3 mm. Then the cellulose sponge was dried and compressed in the thickness direction at 130°C to be a compressed cellulose sponge. The compressed cellulose sponge had a thickness of 0.3 mm (maximum value), and the compression rate was 10.0%.
The above-described compressed cellulose sponge was rolled into a bar shape, in which the lateral direction thereof was the length direction, and the longitudinal direction thereof was the winding direction. Then, the cellulose sponge was placed into a tube-shaped resin mold having a C-shaped cross section and the inner diameter of $\phi$11 mm. Thereafter, the sponge was dried as in Example 2 in order to obtain a roll-shaped retractor. The roll-shaped retractor had a diameter of $\phi$11.0 mm (maximum value) when removed from the resin mold, and its expanding ratio was 42 times in the winding direction, and its exterior shape was a roll shape.

(Example 16)

The block-shaped cellulose sponge was cut and punched to produce a sheet-shaped cellulose sponge having a lateral length of 150 mm × longitudinal length of 180 mm × thickness of 5 mm. Then the cellulose sponge was dried and compressed in the thickness direction at 130°C to be a compressed cellulose sponge. The compressed cellulose sponge had a thickness of 0.5 mm (maximum value), and the compression rate was 10.0%.
The above-described compressed cellulose sponge was rolled into a bar shape, in which the lateral direction thereof was the length direction, and the longitudinal direction thereof was the winding direction. Then, the cellulose sponge was placed into a tube-shaped resin mold having a C-shaped cross section and the inner diameter of $\phi$9 mm. Thereafter, the sponge was dried as in Example 2 in order to obtain a roll-shaped retractor. The roll-shaped retractor had a diameter of $\phi$10.1 mm (maximum value) when removed from the resin mold, and its expanding ratio was 18 times in the winding direction, and its exterior shape was a roll shape.

(Comparative Example 1)

The block-shaped cellulose sponge was sliced and punched to produce a sheet-shaped cellulose sponge having a lateral length of 8 mm × longitudinal length of 300 mm × thickness of 80 mm. The sheet-shaped cellulose sponge was dried and compressed in the thickness direction at 130°C to be a compressed cellulose sponge. The compressed cellulose sponge had a thickness of 8 mm (maximum value), the compression rate was 10.0%, and the length of diagonal line of the compressed cross section (8 mm in the lateral direction $\times$ 8 mm in the compression direction) was 11.3 mm. Its expanding ratio was 10 times in the thickness direction, and its exterior shape was a square pole shape.

[Table 1]

| | Sheet-shaped cellulose sponge | | | | | Properties of compressed cellulose sponge | | | Conditions when rolling cellulose sponge | | | Dying condition for roll-shaped cellulose sponge in resin mold | | Properties of roll-shaped retractor | | | |
|---|---|---|---|---|---|---|---|---|---|---|---|---|---|---|---|---|---|
| | Size (mm) | | | Dried or not | Compressed or not | Thickness (mm) | | Compression rate (%) | Winding direction | Resin mold tube | Diameter of resin mold (mm) | Temperature (°C) | Time (Hr) | Roll diameter after removed from resin mold (mm) | | Expanding ratio in winding direction (time) | Exterior shape |
| | Lateral direction | Longitudinal direction | Thickness direction | | | Average value | Maximum value | | | | | | | Average value | Maximum value | | |
| Example 1 | 120 | 180 | 3 | Dried | Not compressed | - | - | - | Lateral direction | C-shaped circular form Arc-shaped circular form | 9 | 60 | 1 | 10.5 | 10.8 | 11 | Roll shape |
| Example 2 | 120 | 180 | 7 | Dried | Compressed | 0.5 | 0.6 | 8.6 | Lateral direction | C-shaped circular form Arc-shaped circular form | 9 | 60 | 1 | 9.5 | 9.7 | 12 | Roll shape |
| Example 3 | 120 | 180 | 5 | Dried | Compressed | 0.4 | 0.5 | 10.0 | Lateral direction | C-shaped circular form Arc-shaped circular form | 9 | 60 | 1 | 10.1 | 10.3 | 12 | Roll shape |

(continued)

| | Sheet-shaped cellulose sponge | | | | | Properties of compressed cellulose sponge | | | Conditions when rolling cellulose sponge | | | Dying condition for roll-shaped cellulose sponge in resin mold | | Properties of roll-shaped retractor | | | |
| | Size (mm) | | | Dried or not | Compressed or not | Thickness (mm) | | Compression rate (%) | Winding direction | Resin mold tube | Diameter of resin mold (mm) | Temperature (°C) | Time (Hr) | Roll diameter after removed from resin mold (mm) | | Expanding ratio in winding direction (time) | Exterior shape |
| | Lateral direction | Longitudinal direction | Thickness direction | | | Average value | Maximum value | | | | | | | Average value | Maximum value | | |
|---|---|---|---|---|---|---|---|---|---|---|---|---|---|---|---|---|---|
| Example 4 | 120 | 300 | 7 | Dried | Compressed | 0.5 | 0.6 | 8.6 | Lateral direction | C-shaped circular form Arc-shaped circular form | 9 | 60 | 1 | 10.0 | 10.1 | 12 | Roll shape |
| Example 5 | 120 | 300 | 5 | Dried | Compressed | 0.4 | 0.5 | 10.0 | Lateral direction | C-shaped circular form Arc-shaped circular form | 9 | 60 | 1 | 10.6 | 10.9 | 11 | Roll shape |
| Example 6 | 150 | 180 | 5 | Dried | Compressed | 0.4 | 0.5 | 10.0 | Lateral direction | C-shaped circular form Arc-shaped circular form | 9 | 60 | 1 | 9.8 | 10.0 | 15 | Roll shape |

| | Sheet-shaped cellulose sponge | | | | | Properties of compressed cellulose sponge | | | Conditions when rolling cellulose sponge | | | Dying condition for roll-shaped cellulose sponge in resin mold | | Properties of roll-shaped retractor | | | |
|---|---|---|---|---|---|---|---|---|---|---|---|---|---|---|---|---|---|
| | Size (mm) | | | Dried or not | Compressed or not | Thickness (mm) | | Compression rate (%) | Winding direction | Resin mold tube | Diameter of resin mold (mm) | Temperature (°C) | Time (Hr) | Roll diameter after removed from resin mold (mm) | | Expanding ratio in winding direction (time) | Exterior shape |
| | Lateral direction | Longitudinal direction | Thickness direction | | | Average value | Maximum value | | | | | | | Average value | Maximum value | | |
| Example 7 | 100 | 300 | 7 | Dried | Compressed | 0.5 | 0.6 | 8.6 | Lateral direction | C-shaped circular form Arc-shaped circular form | 9 | 60 | 1 | 10.0 | 10.0 | 10 | Roll shape |
| Example 8 | 80 | 250 | 9 | Dried | Compressed | 0.6 | 0.6 | 6.7 | Lateral direction | C-shaped circular form Arc-shaped circular form | 9 | 60 | 1 | 10.3 | 10.6 | 8 | Roll shape |
| Example 9 | 120 | 180 | 7 | Dried | Compressed | 0.5 | 0.6 | 8.6 | Lateral direction | C-shaped circular form | 9 | 60 | 1 | 10.2 | 10.4 | 12 | Roll shape |
| Example 10 | 120 | 180 | 7 | Dried | Compressed | 0.5 | 0.6 | 8.6 | Lateral direction | Circularly-shaped form | 9 | 60 | 1 | 9.8 | 9.8 | 12 | Roll shape |

| | Sheet-shaped cellulose sponge | | | | | Properties of compressed cellulose sponge | | | Conditions when rolling cellulose sponge | | | Dying condition for roll-shaped cellulose sponge in resin mold | | Properties of roll-shaped retractor | | | |
|---|---|---|---|---|---|---|---|---|---|---|---|---|---|---|---|---|---|
| | Size (mm) | | | Dried or not | Compressed or not | Thickness (mm) | | Compression rate (%) | Winding direction | Resin mold tube | Diameter of resin mold (mm) | Temperature (°C) | Time (Hr) | Roll diameter after removed from resin mold (mm) | | Expanding ratio in winding direction (time) | Exterior shape |
| | Lateral direction | Longitudinal direction | Thickness direction | | | Average value | Maximum value | | | | | | | Average value | Maximum value | | |
| Example 11 | 120 | 180 | 5 | Dried | Compressed | 0.4 | 05 | 10.0 | Lateral direction | C-shaped circular form Arc-shaped circular form | 8 | 60 | 1 | 8.6 | 8.7 | 14 | Roll shape |
| Example 12 | 100 | 300 | 7 | Dried | Compressed | 0.5 | 0.6 | 8.6 | Lateral direction | C-shaped circular form Arc-shaped circular form | 8 | 60 | 1 | 8.5 | 8.6 | 12 | Roll shape |
| Example 13 | 100 | 150 | 5 | Dried | Compressed | 0.4 | 0.5 | 10.0 | Lateral direction | C-shaped circular form Arc-shaped circular form | 7 | 60 | 1 | 9.3 | 10.0 | 10 | Roll shape |

(continued)

| | Sheet-shaped cellulose sponge | | | | | Properties of compressed cellulose sponge | | | Conditions when rolling cellulose sponge | | | Dying condition for roll-shaped cellulose sponge in resin mold | | Properties of roll-shaped retractor | | | |
|---|---|---|---|---|---|---|---|---|---|---|---|---|---|---|---|---|---|
| | Size (mm) | | | Dried or not | Compressed or not | Thickness (mm) | | Compression rate (%) | Winding direction | Resin mold tube | Diameter of resin mold (mm) | Temperature (°C) | Time (Hr) | Roll diameter after removed from resin mold (mm) | | Expanding ratio in winding direction (time) | Exterior shape |
| | Lateral direction | Longitudinal direction | Thickness direction | | | Average value | Maximum value | | | | | | | Average value | Maximum value | | |
| Example 14 | 120 | 180 | 3 | Dried | Compressed | 0.3 | 0.3 | 10.0 | Lateral direction | C-shaped circular form Arc-shaped circular form | 4 | 60 | 1 | 4.5 | 4.8 | 25 | Roll shape |
| Example 15 | 120 | 460 | 3 | Dried | Compressed | 0.3 | 03 | 10.0 | Longitudinal direction | C-shaped circular form Arc-shaped circular form | 11 | 60 | 1 | 11.0 | 11.0 | 42 | Roll shape |
| Example 16 | 150 | 180 | 5 | Dried | Compressed | 0.4 | 05 | 10.0 | Longitudinal direction | C-shaped circular form Arc-shaped circular form | 9 | 60 | 1 | 9.6 | 10.1 | 18 | Roll shape |

15

[Table 2]

| | Sheet-shaped cellulose sponge | | | | | Properties of compressed cellulose sponge | | | | | |
| | Size (mm) | | | Dried or not | Compressed or not | Thickness (mm) | | Compression rate (%) | Length of diagonal line (mm) | Expanding ratio in thickness direction (time) | Exterior shape |
| | Lateral direction | Longitudinal direction | Thickness direction | | | Average value | Maximum value | | | | |
| Comparative Example 1 | 8 | 300 | 80 | Dried | Compressed | 8 | 8 | 10.0 | 11.3 | 10 | Square pole shape |

As described above, the retractor having a small cross section area yet having a wide expandability could be obtained in Example. It is understood that the sizes after expanding can be varied among the retractors which have a similar roll diameter. As a result, even when a small opening is used for inserting a retractor into a patient's body for the purpose of reducing burdens on a patient, it is possible to prepare a retractor which expands into a disable size in a body cavity, and effectively protect, lift or retract organs around the target organ. On the contrary, it is understood that the retractor of Comparative Example can expand only to the extent of the compression with respect to the cross section, and thus, the size of the retractor (the area after expanding) is limited by the size of the opening.

It is noted that the properties of the above-described compressed cellulose sponges and roll-shaped retractors obtained in Examples and Comparative Example were determined by the following method.

[Thickness of compressed cellulose sponge]

The thickness of planar portion of the compressed cellulose sponge was measured at four points by using a vernier caliper, and the averaged and maximum values were calculated. 3 to 5 compressed cellulose sponges were prepared.

[Compression rate of compressed cellulose sponge]

Compression rate was calculated by the following equation, in which TB represents the maximum value of the thickness of the compressed cellulose sponge, and TA represents the thickness of the uncompressed sheet-shaped cellulose sponge. Measurement values from 3 to 5 samples were averaged.

$$\text{Compression rate } (\%) = (\text{TB} / \text{TA}) \times 100$$

[Length of diagonal line of compressed cellulose sponge]

The diagonal line of compressed cross section of the compressed cellulose sponge (Comparative Example 1) was measured by using a vernier caliper, and the maximum value was calculated. 3 to 5 compressed cellulose sponges were prepared.

[Exterior shape of compressed cellulose sponge]

The exterior shape of the compressed cellulose sponge was visually observed.

[Diameter of roll-shaped retractor]

The diameter of the roll-shaped retractor was measured at three points, i.e., both ends and the midpoint, by using a vernier caliper, and the averaged and maximum values were calculated. 3 to 5 roll-shaped retractors were prepared.

[Expanding ratio of roll-shaped retractor in winding direction]

Expanding ratio was calculated from the following equation, in which Dmax represents the maximum value of roll diameter of the roll-shaped retractor, and W represents a length of the punched sheet-shaped cellulose sponge in the winding direction. Measurement values from 3 to 5 samples were averaged.

$$\text{Expanding ratio in the winding direction } (\text{time}) = \text{W} / \text{Dmax}$$

[Exterior shape of roll-shaped retractor]

The exterior shape of the roll-shaped retractor was visually observed.

The present invention was specifically described above in connection with the exemplary embodiments of retractor used with an endoscope, but the retractor of the present invention is not limited to these specific examples and may be implemented in various ways. For example, the retractor of the present invention may also be suitable for use in abdominal surgery, pelvic surgery and the like.

DESCRIPTION OF REFERENCE SIGNS

10    Retractor
D     Diameter of retractor
L     Length of retractor
W     Length of widthwise direction (Length of winding direction)

**Claims**

1.  A surgical retractor comprising a liquid-absorbing and swelling material, wherein
    the liquid-absorbing and swelling material is formed in a roll shape, and the roll-shaped liquid-absorbing and swelling
    material absorbs liquid in a body cavity to swell and expand into a planar shape.

2.  The retractor according to claim 1, wherein the roll-shaped liquid-absorbing and swelling material is formed by rolling
    a sheet-shaped liquid-absorbing and swelling material into a roll shape.

3.  The retractor according to claim 2, wherein the sheet-shaped liquid-absorbing and swelling material is compressed
    in the thickness direction.

4.  The retractor according to any one of claims 1 to 3, wherein the roll-shaped liquid-absorbing and swelling material
    expands, in a roll direction, by 8 to 42 times the diameter of the roll.

5.  The retractor according to any one of claims 1 to 4, wherein the roll-shaped liquid-absorbing and swelling material
    lifts and retracts an organ in a vicinity of a target.

FIG. 1A

FIG. 1B

FIG. 1C

FIG. 2

**INTERNATIONAL SEARCH REPORT**

| International application No. |
| --- |
| PCT/JP2015/064042 |

A. CLASSIFICATION OF SUBJECT MATTER
*A61B17/02*(2006.01)i

According to International Patent Classification (IPC) or to both national classification and IPC

B. FIELDS SEARCHED

Minimum documentation searched (classification system followed by classification symbols)
A61B17/02

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | WO 2011/111703 A1  (Kawamoto Corp.), 15 September 2011 (15.09.2011), entire text; all drawings & JP 5313394 B           & EP 2545858 A1 | 1–5 |
| Y | JP 2011-120952 A  (Nippon Medical School), 23 June 2011 (23.06.2011), entire text; all drawings & US 2010/0286474 A1    & WO 2009/084688 A1 & EP 2233101 A1 | 1–5 |
| A | WO 2010/024244 A1  (Kawamoto Corp.), 04 March 2010 (04.03.2010), entire text; all drawings & JP 5128672 B           & EP 2332469 A1 | 1–5 |

| ☒ Further documents are listed in the continuation of Box C. | ☐ See patent family annex. |

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 31 July 2015 (31.07.15) | Date of mailing of the international search report 11 August 2015 (11.08.15) |
| --- | --- |
| Name and mailing address of the ISA/ Japan Patent Office 3-4-3,Kasumigaseki,Chiyoda-ku, Tokyo 100-8915,Japan | Authorized officer Telephone No. |

Form PCT/ISA/210 (second sheet) (July 2009)

**EP 3 146 906 A1**

**INTERNATIONAL SEARCH REPORT**

International application No.

PCT/JP2015/064042

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-089900 A (Toray Fine Chemicals Co., Ltd.), 30 April 2009 (30.04.2009), entire text; all drawings (Family: none) | 1-5 |
| A | US 3961629 A (Ferdinand Joesph RICHTER), 08 June 1976 (08.06.1976), entire text; all drawings (Family: none) | 1-5 |

Form PCT/ISA/210 (continuation of second sheet) (July 2009)

22

**EP 3 146 906 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003164459 A
- JP 2005253916 A
- JP 5128672 B
- JP 3520511 B